Europäisches Patentamt

European Patent Office

Office européen des brevets

(11) Publication number: **0 189 253**
**A2**

# (12) EUROPEAN PATENT APPLICATION

(21) Application number: 86300117.8

(22) Date of filing: 09.01.86

(51) Int. Cl.⁴: **A 61 F 2/38**

(30) Priority: 18.01.85 US 692595

(43) Date of publication of application: 30.07.86
Bulletin 86/31

(84) Designated Contracting States: **AT BE CH DE FR GB IT LI LU NL SE**

(71) Applicant: **Pfizer Hospital Products Group, Inc., 235 East 42nd Street, New York New York 10017 (US)**

(72) Inventor: **Walker, Peter Stanley, 15, Hallett Hill Road, Weston Massachusetts (US)**
Inventor: **Miegel, Robert Ernst, 77, Park Street, Brookline Massachusetts (US)**
Inventor: **McKay, William Francis, Bldg. D., Apt. 6B Hensyn Village, Budd Lake New Jersey (US)**
Inventor: **Ewald, Frederick C., 4, Black Oak Road, Weston Massachusetts (US)**

(74) Representative: **Wood, David John, Pfizer Limited Ramsgate Road, Sandwich Kent CT13 9NJ (GB)**

(54) **Press fit knee prosthesis and instrumentation.**

(57) A knee prosthesis for press fit implantation without fixation cement, including a femoral component (10) in which the dual bearing members (12, 14) each have a distal fixation surface (22, 28) which is curvilinear and concave when viewed in sagittal planes. A novel cutting jig (48) and saw blade (98) for preparing the distal femur for implantation of the femoral component are also disclosed. The fixation post (32, 44) of both the femoral and tibial components of the prosthesis is vane shaped in cross section, and the textured fixation surfaces of the components may include a layer (46) of biocompatible felt material for distributing stresses between the component and the prepared bone member.

PC 6898

PRESS FIT KNEE PROSTHESIS
AND INSTRUMENTATION

This invention concerns a knee prosthesis for insertion on the distal femur and proximal tibia without the use of fixation cement.

Numerous knee replacements have been proposed to stabilize a degenerated knee. Most of the prostheses used today for this purpose are of the condylar replacement type, replacing femoral and tibial surfaces, attached to the bone using acrylic cement. With such devices, the bones are generally prepared by making planar cuts, while the alignments are achieved using special guides. Occasional failure of these devices, generally through loosening, has been attributed to such aspects as poor cement technique, incorrect component alignment, insufficient component surface area and excessive femoral-tibial constraint.

More recently, non-cemented knees using such techniques as porous ingrowth and adjunct fixation have been implanted. While these prostheses, like the cemented type, have met with considerable success, the need still exists for a prosthetic knee of improved stability.

It is therefore the object of the present invention to provide such a knee prosthesis of improved stability and instrumentation for its implantation.

The desired objective is accomplished with a prosthetic knee joint in which the femoral component comprises a spaced pair of bearing members adapted for mutual articulation with an associated tibial component, and a bridging portion joining the bearing members, each of the bearing members having joined anterior, distal and posterior fixation surfaces adapted to fit against a surgically prepared distal end of a femur when the femoral component is implanted, the anterior, distal and posterior fixation surfaces of the bearing members being respectively coincident when viewed in sagittal planes, with at least the distal fixation surface of the bearing members being curvilinear and concave when viewed in sagittal planes. Preferably, the anterior and posterior fixation surfaces of the bearing members are planar.

A cutting jig for use in the preparation of the distal end of a femur prior to implantation of the femoral prosthesis comprises a pair of rail members each having a convex curvilinear edge thereon; means for retaining the rail members in fixed spaced relationship; and means on the rail members for temporarily securing the jig to the distal end of the femur, the curvilinear edges of the rail members being coincident when the jig is secured to the femur and viewed in sagittal planes. Preferably, the cutting jig includes means for positioning the jig on the femur and the curvilinear edges are in the form of a circular arc with an angle of rotation of at least about 45°. A saw blade for use with the cutting jig has a cutting edge with spaced cutting teeth along at least a portion of its length, and a curvilinear cross section congruent with the curvilinear edges of the cutting jig.

A method of preparing the distal end of a femur prior to implantation of the femoral prosthesis comprises the steps of securing a cutting jig having a pair of convex curvilinear cutting edges thereon to the distal end of the femur after proper alignment therewith; making a transverse distal femoral cut in a surface defined by the curvilinear edges; removing the cutting jig; and making the remaining cuts on the distal end of the femur.

The knee prosthesis comprises the femoral component and a tibial component, the tibial component also having a fixation surface, adapted to fit against a surgically prepared proximal tibia when the prosthesis is implanted. At least a portion of the textured fixation surfaces may comprise a layer of biocompatible felt material adapted to be held by a press fit without use of an adhesive and to distribute stresses between the component and the prepared bone member. Preferably, the components each have a fixation post adapted to be secured within the intramedullary canal of the respective bone member, the fixation post of at least one component being vane shaped in cross section throughout a substantial portion of its length.

The above and other objects, features and advantages of the present invention will be apparent from the following detailed description of a preferred embodiment thereof in conjunction with the accompanying drawings wherein like reference numerals indicate like structure throughout the several views.

FIG. 1 is a top plan view of a femoral component suitable for use in the present invention;

FIG. 2 is a rear elevational view partly in cross section taken through FIG. 1 along the line 2-2;

FIG. 3 is a side elevational view in cross section taken through FIG. 1 along the line 3-3;

FIG. 4 is a cross-sectional view taken through FIG. 3 along the line 4-4;

FIGS. 5-7 are front elevational, side elevational and bottom plan views, respectively, of a tibial component suitable for articulation with a femoral component of the present invention;

FIG. 8 is a front elevational view of a cutting jig suitable for use in the preparation the distal end of a femur prior to implantation of a femoral component prosthesis of the present invention, with its locator when rotated downwardly shown in phantom outline;

FIG. 9 is a left side elevational view of the cutting jig of FIG. 8;

FIG. 10 is a front elevational view of a cutting block suitable for use in making additional distal femoral cuts following use of the cutting jig of FIGS. 8 and 9;

FIG. 11 is a cross-sectional view taken through FIG. 10 along the line 11-11;

FIG. 12 is a side elevational view of a saw blade suitable for use in conjunction with the cutting jig of FIGS. 8 and 9;

FIG. 13 is a cross-sectional view taken through FIG. 12 along the line 13-13; and

FIGS. 14-19 are views illustrating the preparation of the distal end of a patient's femur for the implantation of the femoral component of FIGS. 1-4.

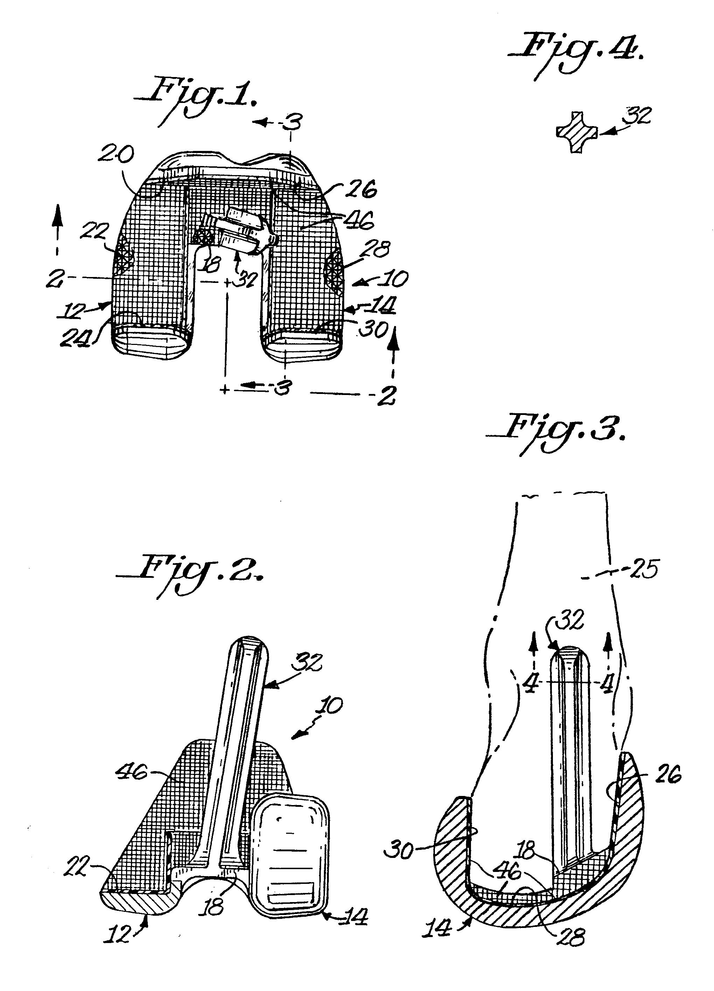

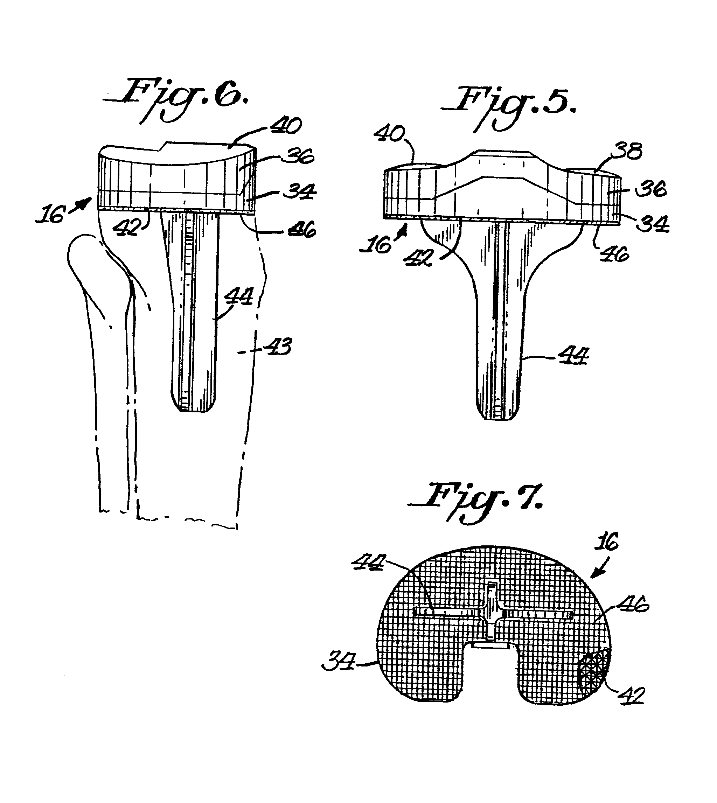

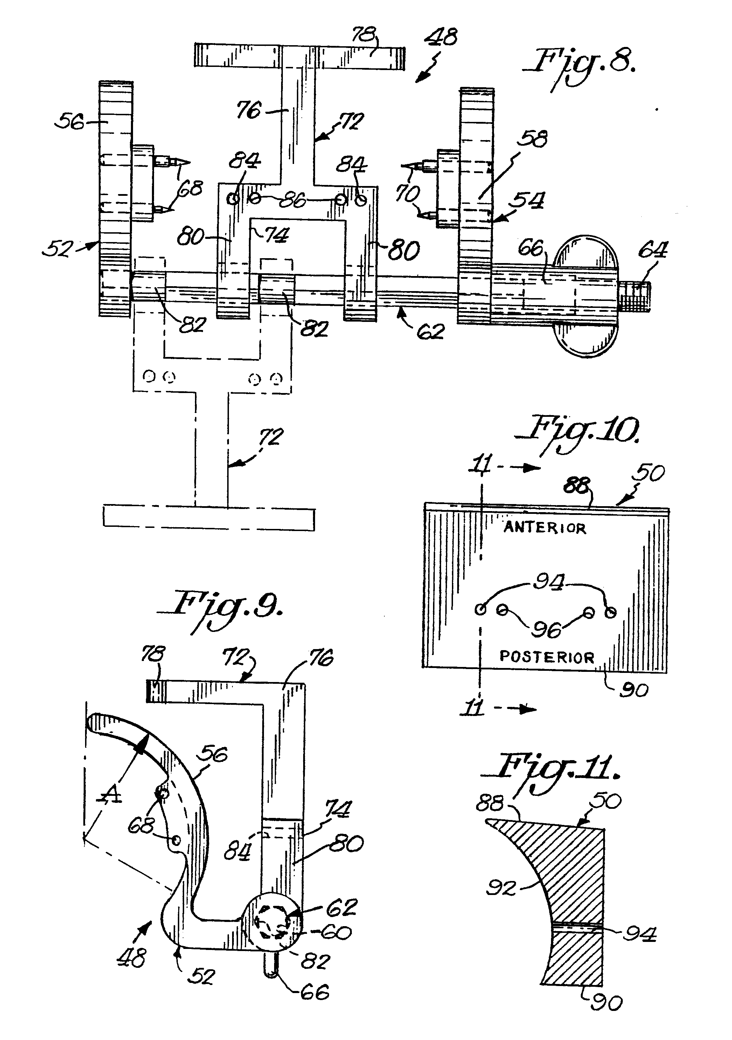

A femoral component 10 (right knee) suitable for use in the present invention is shown in FIGS. 1 to 4.

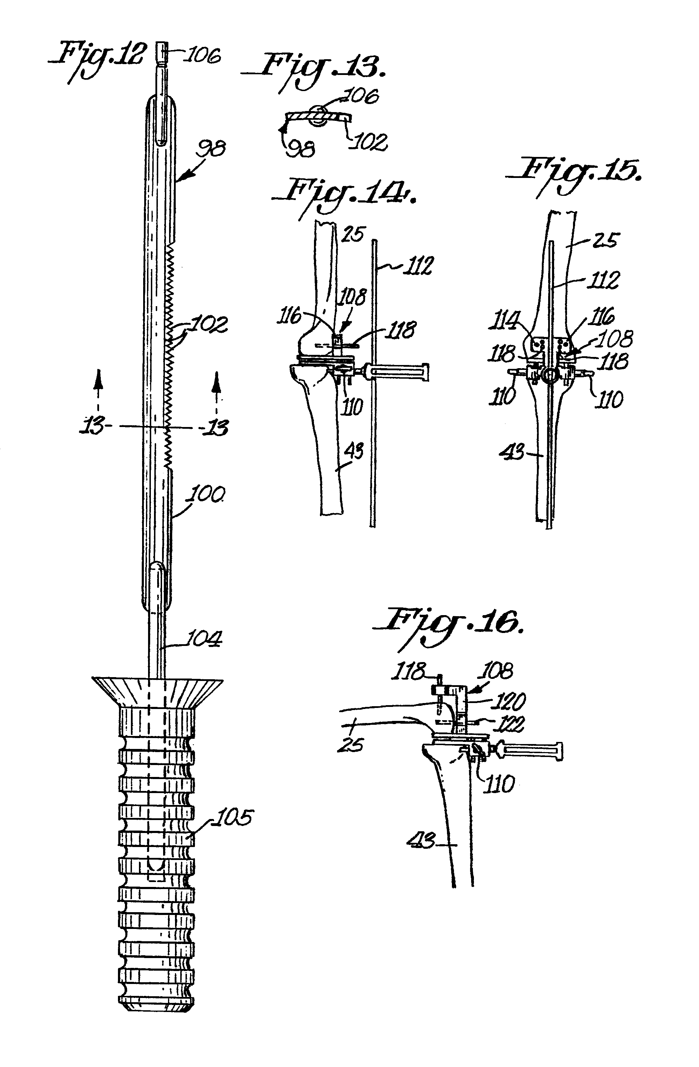

Femoral component 10 comprises a spaced pair of bearing members 12,14 adapted for mutual articulation with an associated tibial component 16, shown in FIGS. 5 to 7, and a bridging portion 18 joining the bearing members 12,14. Bearing member 12 has an anterior fixation surface 20, distal fixation surface 22 and posterior fixation surface 24 adapted to fit against a surgically prepared distal femur 25, shown in phantom outline in FIG. 3, when femoral component 10 is implanted. Likewise, bearing member 14 has an anterior fixation surface 26, distal fixation surface 28 and posterior fixation surface 30. Anterior fixation surfaces 20 and 26 are coincident when viewed in sagittal planes, as are distal fixation surfaces 22 and 28 and posterior fixation surfaces 24 and 30. Anterior fixation surfaces 20,26 and posterior fixation surfaces 24,30 are planar, while distal fixation surfaces 22,28 are cylindrical, appearing as circular arcs when viewed in sagittal planes, being concentric with the respective bearing surfaces of bearing members 12,14. Such curvilinear configuration for distal fixation surfaces 22,28 requires less removal of bone during preparation of distal femur 25 for implantation of femoral component 10, discussed hereinafter, than does the commonly used planar configuration formed by connecting the bases of surfaces 20 with 24 and 26 with 30. Retention of the additional denser, stronger bone stock on distal femur 25 provides a much better foundation for supporting femoral component 10, while the coincident, cylindrical shape of surfaces 22,28 permits a facile method for their preparation.

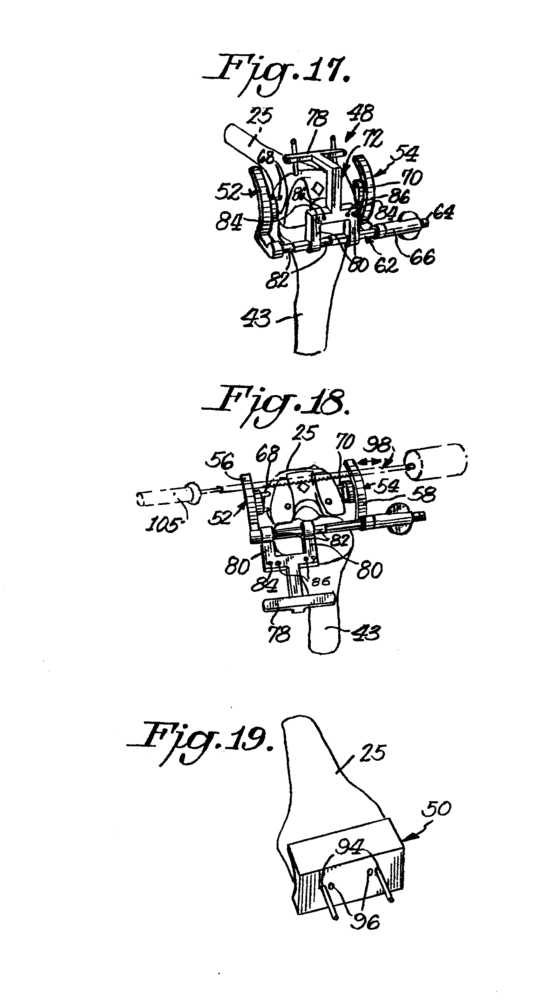

Femoral component 10 has a femoral fixation post 32 attached to bridging portion 18 and adapted to be secured within the intramedullary canal of distal femur 25. Femoral fixation post 32 is vane shaped, as shown in FIG. 4, throughout its length. The proximal end of post 32 is relieved anteriorly to conform with the shape of the intramedullary canal. The entire femoral component is fabricated from biocompatible metal such as Vitallium$^R$.

The associated tibial component 16 is shown in FIGS. 5 to 7. Tibial component 16 comprises a tibial tray 34, of biocompatible metal, and a tibial insert 36, of resilient plastic such as ultra high molecular weight polyethylene, with runners 38 and 40 which articulate with femoral bearing members 12 and 14, respectively. Like femoral component 10, tibial component 16 has a fixation surface 42, adapted to fit against a prepared proximal tibia 43, shown in phantom outline in FIG. 6, and includes a tibial fixation post 44, adapted to be secured in the intramedullary canal of proximal tibia 43. The tibial fixation post 44, as best shown in FIG. 7, is vane shaped in cross section throughout its length.

Since both femoral component 10 and tibial component 16 are intended for implantation by press fit and without the use of fixation cement, they include features to insure the continued stability of the implanted prosthesis. Thus, femoral fixation post 32 and tibial fixation post 44 are, as previously indicated, vane shaped in cross section. This configuration not only allows for a neater press fit with less bone removal and reduced prosthesis weight than does the usual solid stem, but also improves the rotational as well as the medial/lateral and anterior/posterior stability of the knee.

Additionally, the femoral fixation surfaces
20,22,24,26,28,30 and tibial fixation surface 42 are
textured. This texturing, which, as shown in FIGS. 1
and 7, may be in the shape of pyramids with an altitude
of about 0.5 mm, provides grip in shear, enhancing the
resistance to sliding and rotation at the prosthesis/
bone interface.

To further enhance the long term fixation of the
implanted prosthesis, a thin layer 46 of biocompatible
compliant or elastic felt material such as unsupported
Dacron$^R$ felt (Catalog No. 019324, Meadox Medical,
Oakland, NJ) may be placed against any or all of the
fixation surfaces 20,22,24,26,28,30,42 so as to be
interposed at the interface between these surfaces and
prepared bone upon implantation of components 10,16.
The compliant nature of layer 46, which is held in
place by direct compression and by the textured nature
of the fixation surfaces, acts to more uniformly distribute the stresses at the interface. In addition,
both fibrous and bone tissue infiltrate its porous
structure, making layer 46 integral with the bone
surface and causing even more biocompatible stress
distribution. Layer 46 normally has a thickness of
about 2 mm which is compressed about 50 percent in use.

A cutting jig 48 and a cutting block 50 suitable
for use during the preparation of distal femur 25 prior
to implantation of femoral prosthesis 10 are shown in
FIGS. 8 to 11.

Cutting jig 48, which is used in making the
transverse distal cut, comprises two rail members 52
and 54 with curvilinear cutting edges 56 and 58,
respectively. Cutting edges 56,58, which are coincident when cutting jig 48 is secured to distal femur 25
and viewed in sagittal planes, are in the form of circular arcs which should have an angle of rotation A
of at least about 45°, preferably, as shown in
FIG. 9, of about 120°. A hexagonal retaining hole 60
through rail member 54 permits rail member 54 to be
slipped onto, and retained in the desired space relationship to rail member 52 by hexagonal retaining bolt
62, rail member 52 being rigidly attached, such as by
welding, to retaining bolt 62. A threaded portion 64
on retaining bolt 62 allows rail member 54 to be
advanced along the longitudinal axis of retaining bolt
62 toward rail member 52 by the action of threaded wing
nut 66. Securing pins 68,68 on rail member 52 and
securing pins 70,70 on rail member 54 secure the
cutting jig 48 to distal femur 25 while the transverse
distal cut is being made.

Cutting jig 48 also includes a jig locator 72 for
positioning the cutting jig 48 on distal femur 25. Jig
locator 72 comprises a body portion 74, connecting arm
76 and locating bar 78. Body portion 74 has at the
lower extremity of each of its legs 80,80 a hexagonal
hole, congruent with the hexagonal cross section of
retaining bolt 62, which allows jig locator 72 to slide
along the longitudinal axis of retaining bolt 62. Two
cylindrical portions 82,82 of retaining bolt 62, spaced
to be congruent with the spacing between legs 80,80 of
body portion 74 and having a cross-sectional diameter
equal to the flat dimension of the hexagonal cross
section of retaining bolt 62, allow jig locator 72 to
rotate around retaining bolt 62 when legs 80,80 are
located over cylindrical portions 82,82, as shown in
phantom outline in FIG. 8. The two sets of jig positioning holes 84,84 and 86,86 and locator bar 78 are
used in conjunction with positioning pins to properly
position cutting jig 48 on distal femur 25.

Cutting block 50, which provides the planes for the anterior and posterior distal cuts of distal femur 25, comprises planar surfaces 88,90 and cylindrical surface 92, cylindrical surface 92 being congruent with the cylindrical surface formed by cutting edges 56,58. The two sets of block positioning holes 94,94 and 96,96 are used in conjunction with positioning pins to position cutting block 50 on distal femur 25 following the transverse distal cut, as explained hereinafter.

A saw blade 98 suitable for making the transverse distal femoral cut is shown in FIGS. 12 and 13. Saw blade 98 has a cross section in the form of a circular arc, as shown in FIG. 13, which is congruent with cutting edges 56,58 of cutting jig 48, and a blade cutting edge 100 with spaced cutting teeth 102 along a portion of its length. Fixedly attached to cutting blade 98 are, at one end, a handle shaft 104 for removable placement of a handle 105 thereon, and, at the other end, a shank 106 for insertion into a reciprocating saw (not shown).

In the implantation of the present prosthesis, the knee is incised and placed in flexion. A standard tibial alignment guide is then placed on proximal tibia 43 and aligned along the longitudinal axis of the tibia in both anterior/posterior and medial/lateral planes. Two drills (or pins) are used to hold the guide on the proximal tibia while an oscillating saw with flat blade resects the top of the proximal tibia. The guide is then removed, leaving the pins intact.

A knee distractor 108 is placed over the two pins in the proximal tibia while the knee is in extension. The medial/lateral ligaments are tightened by adjusting the thumb screws 110 on the distractor to ensure that the joint will be stable when the components are implanted. An alignment rod 112 is placed through the center of the distractor, and should line up over the center of the femoral head and the ankle; if not, soft tissue adjustments, or ligamentous release, are made to the appropriate ligament to restore proper alignment. The alignment rod must also be parallel to the femoral axis in the anterior/posterior plane to insure that the components are in proper flexion/extension rotational placement. Two drills are placed in the anterior face of distal femur 25 through the holes 114 in the distractor head 116 which correspond to the thickness of the tibial component to be implanted (FIGS. 14 and 15).

The distractor head is removed from the pins 118 in the anterior face of the distal femur, and the knee is placed in flexion using a 90° flexion attachment 120 that fits into the distractor head and over the anterior pins. This assures that the knee is at 90° and that femoral component 10 will be at the proper flexion/ extension rotational placement. The ligaments are tightened in flexion by adjusting the thumb screws on the distractor, and two drills are placed in the distal face of the distal femur using the same holes as used in the previous step (FIG. 16). The distractor is then removed, leaving the distal pins 122.

With the knee still in flexion, jig locator 72 is placed on the femur, sliding the distal pins on the distal femur through positioning holes 84,84 or 86,86 on the jig locator until locator bar 78 touches the anterior pins on the distal femur. Retaining bolt 62 with rail members 52,54 is slid into the jig locator and tightened by wing nut 66, thereby driving securing pins 68,70 into the sides of the distal femur (FIG. 17).

The anterior and distal pins are then removed from the distal femur. The jig locator is slid along the retaining bolt until legs 80,80 are over cylindrical portions 82,82, and the jig locator is rotated back away from the distal femur. Cutting blade 98, powered by a reciprocating saw, is run across cutting edges 56,58 to make the distal femoral cut (FIG. 18). The cutting jig is then removed.

The two distal pins are reinserted, and cutting block 50 is slid over the pins using block positioning holes 94,94 or 96,96. The anterior and posterior femoral cuts are then made using a planar reciprocating blade, and the block and pins removed (FIG. 19).

An intercondylar wall slotter is then centered on the prepared distal femoral surface and impacted into the bone, and an osteotome is run down the front face of the slotter to cut away bone for accommodating the connecting portion 18 of the femoral component. A femoral trial is placed on the distal femur, and a femoral stem slotter shaped and slightly smaller than femoral fixation post 32 is inserted into the cruciform slot and impacted into the bone to shape the bone to accommodate the femoral fixation post.

Tibial templates are centered on the flat resected tibial surface. A tibial stem slotter shaped and slightly smaller than tibial fixation post 44 is placed into the cruciform slot in the template and impacted into the bone to shape the bone to accommodate the tibial fixation post.

Trial insertions of the two components are made, the components are inserted, optionally with felt layer 46 in place, the patella is prepared and cemented in usual fashion, and the joint is closed.

0189253

The various aspects of the present invention have been described in detail with reference to certain preferred embodiments thereof.  Reference to these embodiments does not, however, limit the scope of the invention, which is limited only by the scope of the claims.

## CLAIMS

1.  A femoral component (10) for a prosthetic knee joint which comprises a spaced pair of bearing members (12,14) adapted for mutual articulation with an associated tibial component (16) and a bridging portion (18) joining the bearing members (12,14), each of the bearing members (12,14) having joined anterior (20,26), distal (22,28) and posterior (24,30) fixation surfaces adapted to fit against a surgically prepared distal end (25) of a femur when the femoral component (10) is implanted, the anterior (20,26), distal (22,28) and posterior (24,30) fixation surfaces of the bearing members (12,14) being respectively coincident when viewed in sagittal planes, characterized in that at least the distal fixation surface (22,28) of the bearing members (12,14) is curvilinear and concave when viewed in sagittal planes.

2.  The femoral component (10) of claim 1 wherein the anterior (20,26) and posterior (24,30) fixation surfaces of the bearing members (12,14) are planar.

3.  A cutting jig (48) for use in the preparation of the distal end (25) of a femur prior to implantation of a femoral prosthesis, which comprises a pair of rail members (52,54) each having a convex curvilinear edge (56,58) thereon; means (60,62) for retaining the rail members (52,54) in fixed spaced relationship; and means (68,70) on the rail members (52,54) for temporarily securing the jig (48) to the distal end (25) of the femur, the curvilinear edges (56,58) of the rail members (52,54) being coincident when the jig (48) is secured to the femur and viewed in sagittal planes.

4.   The cutting jig (48) of claim 3 including means (72) for positioning the jig (48) on the femur.

5.   The cutting jig (48) of claim 3 wherein the curvilinear edges (56,58) are in the form of a circular arc with an angle of rotation of at least about 45°.

6.   A saw blade (98) for use with the cutting jig (48) of claim 3, the blade (98) having a cutting edge (100) with spaced cutting teeth (102) along at least a portion of its length, and a curvilinear cross section congruent with the curvilinear edges (56,58) of the cutting jig (48).

7.   A method of preparing the distal end (25) of a femur prior to implantation of a femoral prosthesis, which comprises the steps of securing a cutting jig (48) having a pair of convex curvilinear cutting edges (56,58) thereon to the distal end (25) of the femur after proper alignment therewith; making a transverse distal femoral cut in a surface defined by the curvilinear edges (56,58); removing the cutting jig (48); and making the remaining cuts on the distal end (25) of the femur.

Fig.1.

Fig.4.

Fig.2.

Fig.3.

_Fig.6._

40
36
34
46
16
42
44
43

_Fig.5._

40
38
36
34
16
42
46
44

_Fig.7._

16
44
46
34
42

0189253

Fig.8.

Fig.9.

Fig.10.

Fig.11.

Fig.12

Fig.13.

Fig.14.

Fig.15.

Fig.16.

*Fig.17.*

*Fig.18.*

*Fig.19.*